(19) Europäisches Patentamt
European Patent Office
Office européen des brevets

(11) **EP 3 629 263 A1**

(12) **EUROPEAN PATENT APPLICATION**

(43) Date of publication:
**01.04.2020 Bulletin 2020/14**

(51) Int Cl.:
**G06Q 10/06** *(2012.01)*        **G01N 33/00** *(2006.01)*

(21) Application number: **18197001.3**

(22) Date of filing: **26.09.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Valeo Systemes Thermiques-THS**
**78322 Le Mesnil Saint Denis Cedex (FR)**

(72) Inventors:
• **DE PELSEMAEKER, Georges**
  **78322 LE MESNIL SAINT DENIS CEDEX (FR)**
• **CLEMARON, Laetitia**
  **78322 LE MESNIL SAINT-DENIS CEDEX (FR)**
• **DEVEYCX, Marion**
  **78322 LE MESNIL SAINT-DENIS (FR)**

(74) Representative: **Metz, Gaëlle**
  **Valeo Systèmes Thermiques**
  **8, rue Louis Lormand**
  **CS 80517 La Verrière**
  **78322 Le Mesnil Saint Denis Cedex (FR)**

(54)     **A SYSTEM ESTIMATING AIR QUALITY INDEX**

(57)     A system for estimating Air Quality Index includes a geo-location module, at least one onboard sensor, a pollution map module and a processing module. The geo-location module detects location of vehicle. The at least one onboard sensor measures at least one of at least one air quality parameter and pollutant concentration at different locations and corresponding times. The pollution map module determines digital pollution map concentration of at least one air quality parameter and at least one pollutant at corresponding same locations and times. The processing module computes a correlation factor and a sensitivity factor. The processing module computes a corrected estimate of concentration of at least one pollutant using digital pollution map concentration and at least one of correlation factor and sensitivity factor. The processing module computes the Air Quality Index based on corrected estimate of concentration and measurement of at least one of air quality parameter and pollutant.

FIG. 1

EP 3 629 263 A1

**Description**

Air pollution is a serious concern for people because of its harmful effects to health - both in physical and psychological sense. Passengers travelling in vehicles are especially at risk, as the vehicles are part of traffic existing in urbanized areas, which tend to have higher concentration of air pollution and higher levels of pollutant gases and particles.

Pollution levels are monitored by government agencies and private entities. The measurements are often provided in form of so called pollution maps. For example, a layer of visually represented information is added to a classic map, so that pollution concentration within a specific area or region can be determined and evaluated. Such maps usually have a set time-resolution, which means that the information is updated for example once an hour or once a day. Pollution map data is obtained for example by measuring pollution with a number of measuring stations or pollution measurement devices of a fixed position.

For determining the Air Quality Index, particulate matter concentration, particularly, PM2.5 and PM10 concentration along with concentration of pollutants such as gases, for example but not limited to, Carbon monoxide (CO), Nitrogen-dioxide ($NO_2$), Sulfur-di-oxide ($SO_2$) and Ozone gas ($O_3$) are required to be determined.

By Air Quality Index, it is meant that any kind of index relation to pollution level.

Generally, the Air Quality Index is determined based on particulate matter PM2.5 and PM10 concentrations in the air determined using PM2.5 and PM10 sensors and concentration of other pollutants, particularly gases such as Carbon monoxide (CO), Nitrogen-di-oxide ($NO_2$), Sulfur-di-oxide ($SO_2$) and Ozone gas ($O_3$) in the air determined using respective pollutant sensors such as gas sensors. Although, the gas sensors used for determining concentration of Carbon monoxide (CO), Nitrogen-di-oxide ($NO_2$), Sulfur-di-oxide ($SO_2$) and Ozone gas ($O_3$) in the air are accurate, however, the gas sensors are not always adapted to automotive constraints. Alternatively, the concentration of pollutants such as Carbon monoxide (CO), Nitrogen-di-oxide ($NO_2$), Sulfur-di-oxide ($SO_2$) and Ozone gas ($O_3$) along with concentration of the particulate matters, PM2.5 and PM10 in the air are determined based on digital pollution map stored in a remote server. However, the digital pollution map concentration of the pollutants such as the above mentioned gases and particulate matter conventionally determined based on the digital pollution maps are based on pollution forecast based on emission sources data, fixed station sensors data, weather data and city topography which implies it is not taking into account particular events in real-time such as more polluting vehicles or a car accident which is implying traffic congestion.

Accordingly, there is a need for a system for determining Air quality index that is taking into account particular real time events to better protect passengers inside the vehicle. Further, there is a need for a system for determining Air quality index that are reliable, requires less maintenance and that can be used in stringent vehicle environment conditions. Furthermore, there is a need for a system for determining Air quality index that indirectly improves performance and efficiency of the air conditioning unit by facilitating accurate detection of the Air quality index for accurate control of the re-circulation flap and all other de-pollution devices such as for example air purifiers.

An object of the present invention is to provide a system for determining Air quality index that obviates the drawbacks associated with conventional system for determining Air Quality Index based on digital pollution map concentrations of particulate matter and the other pollutants such as gases from a digital pollution map.

Still another object of the present invention is to provide a system for determining Air quality index based on inputs from pollutant sensors which are implied by particular event such as a wrong estimation of the traffic, these pollution sensors can be PM2.5, PM10 and/ or nitrogen dioxide (NO2) sensors for example.

Yet another object of the present invention is to provide a system for determining Air quality index that are reliable, requires less maintenance and that can be used in stringent vehicle environment conditions.

Another object of the present invention is to provide a system for determining Air quality index that is compact in configuration due to elimination of at least some of the gas sensors and as such can be conveniently packaged in a limited confined space of a vehicle.

Still another object of the present invention is to provide a system for determining Air quality index that utilizes corrected estimates of pollutant concentration that are corrected by considering correlation, sensitivity and tuning based on big data over several segments of travel stored in a digital pollution map, thereby enhancing accuracy of the system.

In the present description, some elements or parameters may be indexed, such as a first element and a second element. In this case, unless stated otherwise, this indexation is only meant to differentiate and name elements which are similar but not identical. No idea of priority should be inferred from such indexation, as these terms may be switched without betraying the invention. Additionally, this indexation does not imply any order in mounting or use of the elements of the invention.

A system for estimating Air Quality Index, hereinafter referred to as "system" is disclosed in accordance with an embodiment of the present disclosure. The system includes a geo-location module, at least one onboard sensor, a pollution map module and a processing module. The geo-location module detects location of a vehicle at any time. The at least one onboard sensor measures at least one air quality parameter and concentration of at least one pollutant in the close vicinity of the vehicle at different locations of the vehicle and corresponding times. The pollution map module is configured with at least one transceiver that transmits location and time data related to the vehicle at the different locations and corresponding times to a remote server and receives digital pollution map concentration of the at least one air quality parameter and digital pollution map concentration of the at least one pollutant in the air at the corresponding same locations and times based on a digital pollution map stored in the remote server. The processing module computes a correlation factor and a sensitivity factor. The processing module further computes a corrected estimate of concentration of the at least one pollutant in the air at the corresponding locations and times by using the digital pollution concentration and the at least one of the correlation factor and the sensitivity factor. The processing module still further computes the Air Quality Index of the air based on corrected estimate of concentration of the at least one pollutant and at least one of actual measurement of the at least one air quality parameter and actual concentration measurement of at least one pollutant measured by the at least one onboard sensor.

Generally, the geo-location module is a geo-location module that is already configured in at least one of an infotainment system disposed in the vehicle, a hand held, portable communication device that is communicably coupled to the vehicle and carried by any of the vehicle occupants.

Specifically, the at least one onboard sensor is at least one outside embedded sensor configured outside the vehicle that measures the at least one of at least one air quality parameter and concentration of at least one pollutant such as gas.

In accordance with an embodiment, the at least one onboard sensor is at least one of particulate matter sensor and/or gas sensor.

Particularly, the pollution map module receives digital pollution map concentration for every available pollutants PM2.5, PM10, Carbon monoxide (CO), Nitrogen dioxide (NO2), Sulfur dioxide (SO2), Ozone (O3), and other pollutants and/or pollens based on big data over several segments of travel from the digital pollution map stored in the remote server.

Generally, the at least one transceiver is a transceiver that is already configured in at least one of the infotainment system of the vehicle and any hand held, portable communication device such as smart phone that is communicably coupled to said vehicle and carried by any of the vehicle occupants.

Generally, the processing module computes the correlation factor based on comparison of the at least one of actual measurement of the at least one air quality parameter and actual concentration measurement of least one pollutant measured by the onboard sensor in a close vicinity of the vehicle at different locations and time to the corresponding digital pollution map concentration of the at least one air quality parameter and the at least one pollutant detected by the pollution map module at the corresponding same locations of the vehicle and the corresponding same times based on big data over several segments of travel from the digital pollution map.

Further, the processing module computes the sensitivity factor based on comparison between actual pollution type detected to digital pollution map based pollution type detected by said pollution map module by referring to big data over several segments of travel from the digital pollution map, also, the processing module further tunes the sensitivity factor based on comparison between the corrected estimate of concentration of the pollutants and digital pollution map concentration of the pollutants based on big data over several segments of travel from digital pollution map.

Typically, at least one transceiver of the pollution map module of the system of all the other vehicles configured therewith transmits the corrected estimate of concentration of the at least one other pollutant in the air and at least one of the actual measurement of the at least one air quality parameter and actual concentration measurement of at least one pollutant measured by the at least one onboard sensor at various different locations of the vehicle and times to dynamically update the digital pollution map and enhance accuracy of the Air Quality Index so determined.

Further, the system for estimating Air Quality Index further includes a display module for displaying the Air Quality Index determined by the system to the vehicle occupants, wherein display is a display configured on at least one of the infotainment system disposed in the vehicle, a hand held, portable communication device that is communicably coupled to the vehicle and carried by any of the vehicle occupants.

A heating ventilation and Air conditioning system is disclosed in accordance with an embodiment of the present invention that controls operation of a re-circulation flap configured upstream of an evaporator of the heating ventilation and Air conditioning system to selectively close at least one of a fresh air supply conduit and a recirculation air supply conduit based on Air Quality Index determined by the system as claimed in any one of the preceding claims, better estimation of the real-time AQI will improve management and control of all others de-pollution devices to better protect cabin.

A method for estimating Air Quality Index includes the steps of detecting location of a vehicle at any time by using a geo-location module, measuring at least one of at least one air quality parameter and at least one pollutant such as gas and/or particulate matter in close vicinity of the vehicle at different locations of the vehicle and corresponding times by using at least one on-board sensor, determining by a pollution map module, digital pollution map concentration of the at least one air quality parameter and digital pollution map concentration of the at least one pollutant in the air at the corresponding same locations and times based on a digital pollution map stored in a remote server, computing by a processing module a correlation factor and a sensitivity factor, further computing by the processing module a corrected estimate of concentration of the at least one pollutant in the air at the corresponding locations and times by using the digital pollution map concentration of the at least one pollutant and at least one of the sensitivity factor and the correlation factor, further computing by the processing module the Air Quality Index of the air based on corrected estimate of concentration of the at least one pollutant and at least one of actual measurement of the at least one air quality parameter and actual concentration measurement of the at least one pollutant measured by the at least one onboard sensor.

Further, the method includes a step of computing the correlation factor based on comparison of the at least one of actual measurement of the at least one air quality parameter and actual concentration measurement of at least one pollutant measured by the onboard sensor in close vicinity of the vehicle at different locations and times to the corresponding digital pollution map concentration of the at least one air quality parameter and digital pollution map concentration of at least one pollutant detected by the pollution map module at the corresponding same locations and times based on big data over several segments of travel from the digital pollution map.

Further, the method includes the step of computing the sensitivity factor based on comparison between actual pollution type detected to digital pollution map based pollution type detected by the pollution map module by referring to big data over several segments of travel from the digital pollution map, the sensitivity factor is further tuned by the processing module based on comparison between the corrected estimate of concentration of the pollutants and digital pollution map concentration of the pollutants based on big data over several segments of travel from digital pollution map.

Generally, the sensitivity factor is either one of a diagonal matrix and a full matrix of $S_{i,j}$, wherein $i,j$ are pollutants for Air Quality Index (AQI) calculation, $S_{i,j}$ are always corrected by machine learning based on the big data.

Still further, the method includes the step of transmitting by at least one transceiver of the pollution map module of all the other vehicles configured with the system, the corrected estimate of concentration of the at least one pollutant in the air and at least one of the actual measurement of the at least one air quality parameter and actual concentration measurement of the at least one pollutant measured by the at least one onboard sensor at the various different locations of the vehicle and times in order to dynamically update the digital pollution map and enhance accuracy of the Air Quality Index (AQI) so determined in close vicinity of the vehicles at different times.

Furthermore, the method includes the step of displaying on a display module the Air Quality Index determined by the system to the vehicle occupants, wherein the display module is a display configured on at least one of an infotainment system disposed in the vehicle, a hand held, portable communication device that is communicably coupled to the vehicle and carried by any of the vehicle occupant.

Other characteristics, details and advantages of the invention can be inferred from the description of the invention hereunder. A more complete appreciation of the invention and many of the attendant advantages thereof will be readily obtained as the same becomes better understood by reference to the following detailed description when considered in connection with the accompanying figures, wherein:

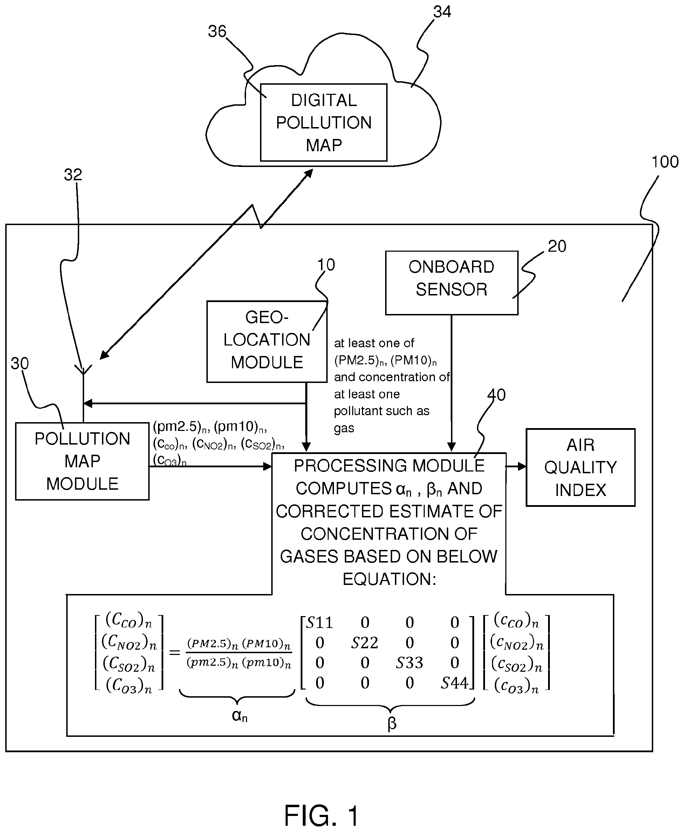

  **FIGURE 1** illustrates a block diagram depicting various elements of a system for estimating Air Quality Index and interaction between the different elements thereof in accordance with an embodiment of the present invention; and

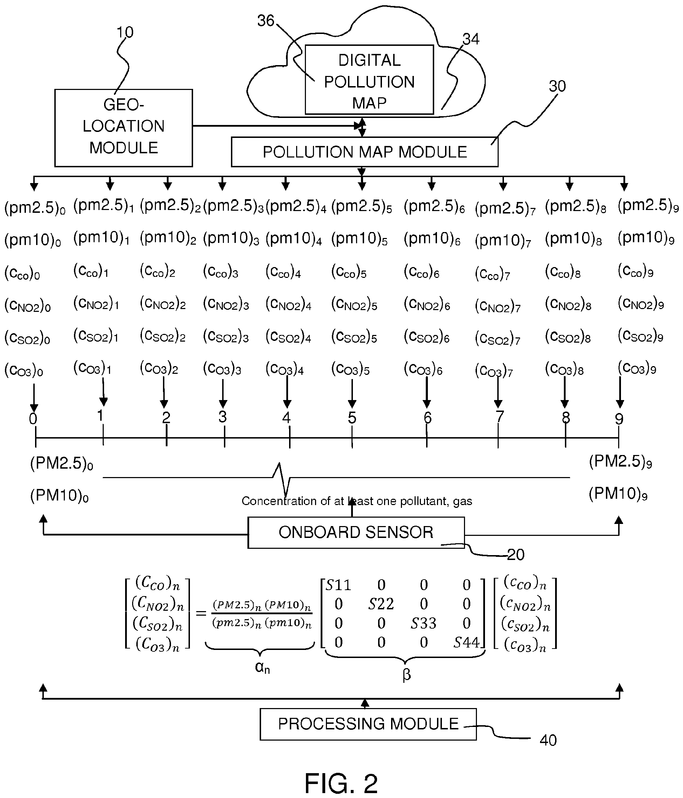

  **FIGURE 2** illustrates a schematic block diagram representation depicting digital pollution map providing digital pollution map concentration of particulate matter and digital pollution map concentration of at least one pollutant at different locations and corresponding times, onboard sensor detecting at least one of particulate matter concentration and concentration of at least one pollutant and the processing module computing a correlation factor, a sensitivity factor and a corrected estimate of concentration of the at least one pollutant.

It must be noted that the figures disclose the invention in a detailed enough way to be implemented, said figures helping to better define the invention if needs be. The invention should however not be limited to the embodiment disclosed in the description.

In the following example, the Air Quality Index (AQI) is determined by using air quality parameters such as particulate matters (PM2.5 or PM10) but the invention is not limited to this specific kind of pollutants and can be applied to other pollutants such as gas for example. Further, apart from measure of the particulate matter concentration, the air quality parameter can be gas concentration, humidity, temperature and the likes.

The present invention envisages a system for estimating Air Quality Index, hereinafter also referred to as "system" to control de-pollution devices. In case of the "system" in accordance with the present invention, the Air Quality Index is determined based on corrected estimate of concentration of at least one pollutant required for calculating the Air Quality Index and further based on inputs from at least one of PM2.5 and PM10 sensors and at least one pollutant sensor. The corrected estimate of concentration of at least one pollutant is corrected by considering correlation, sensitivity and tuning based on big data over several segments of travel. The system of the present invention can also be used for determining any other indicators of air quality associated with the air, not limited to the Air Quality Index and for use in any vehicular or non-vehicular sub-system, that involves determining concentration of particulate matter along with concentration of at least one pollutant such as gases while still limiting number of embedded sensors. Also, the system of the present invention is specifically more useful and economical in case used for determining air quality indicators that require concentration of numerous pollutants such as gases and as such require a large number of gas sensors, as the system of the present invention eliminates at least few of the gas sensors. More specifically, in some cases the "system" of the present invention completely eliminates the gas sensors while in other cases the "system" of the present invention limits the use of the gas sensors.

**FIGURE 1** of the accompanying drawings illustrates a block diagram depicting various elements of a system **100** for estimating Air Quality Index and interaction between the different elements thereof in accordance with an embodiment of the present invention. The system **100** includes a geo-location module **10**, at least one onboard sensor **20**, a pollution map module **30** and a processing module **40**.

The geo-location module **10** detects location of the vehicle at any time. In accordance with one embodiment, the geo-location module **10** is a geo-location module that is configured in at least one of an infotainment system disposed in the vehicle, a hand held, portable communication device that is communicably coupled to the vehicle via a communication network such as for example but not limited to, WIFI, Bluetooth® and the likes and that is carried by any of the vehicle occupants. Specifically, the geo-location module **10** is not essentially dedicated for use as a part of the system **100** only. In accordance with another embodiment, the geo-location module **10** is specifically dedicated for use as a part of the system **100** for estimating Air Quality Index of the present invention. However, the present invention is not limited to any particular configuration of the geo-location module **10** as far as the geo-location module **10** facilitates in detecting location of the vehicle at any time.

The at least one onboard sensor **20** is a sensor that measures at least one air quality parameter and/or at least one pollutant associated with air outside a cabin of the vehicle in close vicinity of the vehicle at different times. Specifically, the at least one onboard sensor **20** is at least one outside embedded sensor configured outside the vehicle that measures the at least one air quality parameter that is linked to particular events which are difficult to estimate before. For example, the at least one onboard sensor **20** is at least one of particulate matter sensor, gas sensor, humidity sensor and temperature sensor and the likes. In accordance with a preferred embodiment of the present invention, the at least one onboard sensor **20** is at least one of PM 2.5 and PM 10 sensor and gas sensor, for example NO2 sensor. However, the present invention is not limited to any particular type, configuration, placement and number of the at least one onboard sensor **20** as far as the at least onboard sensor **20** is capable of accurately measuring/detecting the at least one of at least one air quality parameter and at least one pollutant such as for example gas.

The pollution map module **30** is configured with at least one transceiver **32** that transmits location and time data related to the vehicle at the different locations and corresponding times to a remote server **34** and receives a digital pollution map concentration $(pm2.5)_n$, $(pm10)_n$ of the at least one air quality parameter and digital pollution map concentration $(c_{CO})_n$, $(c_{NO2})_n$, $(c_{SO2})_n$, $(c_{O3})_n$ of the at least one pollutant in the air in the vicinity of the vehicle at the corresponding same locations and times based on a digital pollution map **36** stored in the remote server **34**. Specifically, the pollution map module **30** receives the digital pollution map concentration $(pm2.5)_n$, $(pm10)_n$ of the at least one air quality parameter and the digital pollution map concentration $(c_{CO})_n$, $(c_{NO2})_n$, $(c_{SO2})_n$, $(c_{O3})_n$ of at least one pollutant in the air at the corresponding same locations and times at which the at least one onboard sensor **20** measured the at least one of at least one air quality parameter and at least one pollutant associated with air outside the cabin of the vehicle. The at least one transceiver **32** is a transceiver that is configured in at least one of the infotainment system of the vehicle, any hand held, portable communication device that is communicably coupled to the vehicle via a communication network such as for example, but not limited to, WIFI and Bluetooth® and that is carried by any of the vehicle occupants. Specifically, the at least one transceiver is not essentially dedicated for use as a part of the system **100** only. In accordance with another embodiment, the at least one transceiver **32** is specifically dedicated for use as a part of the system **100**. The at least one transceiver **32** of the pollution map module **30** of the system **100** of all the other vehicles configured therewith, transmits a corrected estimate $(C_{CO})_n$, $(C_{NO2})_n$, $(C_{SO2})_n$, $(C_{O3})_n$ of concentration of the at least one pollutant in the air and at least one of the actual measurement $(PM2.5)_n$, $(PM10)_n$ of the at least one air quality parameter and actual concentration measurement of at least one pollutant measured by the at least one onboard sensor **20** at the various different locations of the vehicle and times in order to dynamically update the digital pollution map **36** and enhance accuracy of the Air Quality Index (AQI) so determined. However, the present invention is not limited to any particular method or device or system and configuration of such method, device and system for determining digital pollution map concentration $(pm2.5)_n$, $(pm10)_n$ of at least one air quality parameter and digital pollution map concentration $(c_{CO})_n$, $(c_{NO2})_n$, $(c_{SO2})_n$, $(c_{O3})_n$ of at least one pollutant in the air at different locations and corresponding times.

The pollution map module **30** receives digital pollution map concentration for every available pollutants PM2.5, PM10, Carbon monoxide (CO), Nitrogen dioxide (NO2), Sulfur dioxide (SO2), Ozone (O3), and other pollutants and/or pollens not limited to the above pollutants based on big data over several segments of travel from the digital pollution map **36** stored in the remote server **34**.

**FIGURE 2** illustrates a schematic block diagram representation depicting the at least one onboard sensor **20** detecting at least one of particulate matter concentration $(PM2.5)_n$ and $(PM10)_n$ and concentration of at least one pollutant for example gases in close vicinity of the vehicle and the digital pollution map **36** providing digital pollution map concen-

tration $(pm2.5)_n$, $(pm10)_n$ of particulate matter and digital pollution map concentration $(c_{CO})_n$, $(c_{NO2})_n$, $(c_{SO2})_n$, $(c_{O3})_n$ of at least one pollutant at corresponding same locations and times and the processing module **40** computing a correlation factor $(\alpha_n)$, a sensitivity factor $(\beta)$ and the corrected estimate $(C_{CO})_n$, $(C_{NO2})_n$, $(C_{SO2})_n$, $(C_{O3})_n$ of concentration of the at least one pollutant.

Specifically, the processing module **40** computes the correlation factor $\alpha_n$ and the sensitivity factor $(\beta)$. The processing module **40** computes the correlation factor $(\alpha_n)$ based on comparison of the at least one of actual measurement $(PM2.5)_n$, $(PM10)_n$ of the at least one air quality parameter and actual concentration measurement of at least one pollutant measured by the onboard sensor **20** at different locations and times to the corresponding digital pollution map concentration $(pm2.5)_n$, $(pm10)_n$ of the same at least one air quality parameter and digital pollution map concentration of the same at least one pollutant detected by the pollution map module **30** at the corresponding same locations and times based on big data over several segments of travel stored in the digital pollution map **36**. More specifically, the processing module **40** compares the at least air quality parameter based on pollution map and measurement at the vehicle location at "t" time to determine the correlation factor $\alpha_n$.

Specifically, the processing module **40** computes the correlation factor $(\alpha_n)$ based on comparison of the actual measurement $(PM2.5)_n$, $(PM10)_n$ of the at least one air quality parameter measured by the onboard sensor **20** at different locations and times to the digital pollution map concentration $(pm2.5)_n$, $(pm10)_n$ of the same at least one air quality parameter detected by the pollution map module **30** at the corresponding same locations and times based on big data over several segments of travel stored in the digital pollution map **36**.

Alternatively, the processing module **40** computes the correlation factor $(\alpha_n)$ based on comparison of the at least one actual measurement of at least one pollutant such as at least one gas at different locations and times to the digital pollution map concentration of the same gas detected by the pollution map module **30** at the corresponding same locations and times based on big data over several segments of travel stored in the digital pollution map **36**. The processing module **40** computes the correlation factor $\alpha_n$ based on the following formula/calculations:

$$\alpha_n = \text{Measured pollutant / Map estimation concentration for same pollutant} = (PM2.5)_n {}^* (PM10)_n / (pm2.5)_n {}^* (pm10)_n$$

Referring to the **FIGURE 2**, the processing module **40** determines the correlation factors $\alpha_0$, $\alpha_1$, $\alpha_2$, $\alpha_3$, $\alpha_4$, $\alpha_5$, $\alpha_6$, $\alpha_7$, $\alpha_8$ and $\alpha_9$, at different locations 0 to 9 as under.

$$\alpha_0 = (PM2.5)_0 {}^* (PM10)_0 / (pm2.5)_0 {}^* (pm10)_0$$

$$\alpha_1 = (PM2.5)_1 {}^* (PM10)_1 / (pm2.5)_1 {}^* (pm10)_1$$

$$\alpha_2 = (PM2.5)_2 {}^* (PM10)_2 / (pm2.5)_2 {}^* (pm10)_2$$

$$\alpha_3 = (PM2.5)_3 {}^* (PM10)_3 / (pm2.5)_3 {}^* (pm10)_3$$

$$\alpha_4 = (PM2.5)_4 {}^* (PM10)_4 / (pm2.5)_4 {}^* (pm10)_4$$

$$\alpha_5 = (PM2.5)_5 {}^* (PM10)_5 / (pm2.5)_5 {}^* (pm10)_5$$

$$\alpha_6 = (PM2.5)_6 {}^* (PM10)_6 / (pm2.5)_6 {}^* (pm10)_6$$

$$\alpha_7 = (PM2.5)_7 {}^* (PM10)_7 / (pm2.5)_7 {}^* (pm10)_7$$

$$\alpha_8 = (PM2.5)_8 * (PM10)_8 / (pm2.5)_8 * (pm10)_8$$

$$\alpha_9 = (PM2.5)_9 * (PM10)_9 / (pm2.5)_9 * (pm10)_8$$

The processing module **40** further computes the sensitivity factor ($\beta$) based on comparison between actual pollution type detected to digital pollution map based pollution type detected by said pollution map module **30** by referring to big data over several segments of travel from the digital pollution map **36**, the processing module **40** further tunes the sensitivity factor based on comparison between the corrected estimate $(C_{CO})_n$, $(C_{NO2})_n$, $(C_{SO2})_n$, $(C_{O3})_n$ of concentration of the pollutants and digital pollution map concentration $(c_{CO})_n$, $(c_{NO2})_n$, $(c_{SO2})_n$, $(c_{O3})_n$ of the gases based on big data over several segments of travel stored the digital pollution map **36**.

The processing module computes the sensitivity factor ($\beta$), based on the following formula/calculations:

$$\beta = \begin{bmatrix} S11 & 0 & 0 & 0 \\ 0 & S22 & 0 & 0 \\ 0 & 0 & S33 & 0 \\ 0 & 0 & 0 & S44 \end{bmatrix}$$

The sensitivity coefficient $S_{i,i}$, where $i = 1, n$ (for n pollutant type) are determined based on comparison between the corrected estimate $(C_{CO})_n$, $(C_{NO2})_n$, $(C_{SO2})_n$, $(C_{O3})_n$ of concentration of the pollutants and digital pollution map concentration $(c_{CO})_n$, $(c_{NO2})_n$, $(c_{SO2})_n$, $(c_{O3})_n$ of the pollutants based on big data over several segments of travel from the digital pollution map **36**

The Sensitivity matrices could be diagonal as the formulas here above or a complete matrices in which all cell $S_{i,j}$ for i and j = 1, n are estimated with the same method. The sensitivity factor ($\beta$) is either one of a diagonal matrix and a full matrix of $S_{i,j}$, wherein i,j are pollutants for Air Quality Index (AQI) calculation, $S_{i,j}$ are always corrected by machine learning based on the big data.

The processing module **40** further computes the corrected estimate $(C_{CO})_n$, $(C_{NO2})_n$, $(C_{SO2})_n$, $(C_{O3})_n$ of concentration of the at least one pollutant in the air in the vicinity of the vehicle at the corresponding locations and times by using the digital pollution map concentration $(c_{CO})_n$, $(c_{NO2})_n$, $(c_{SO2})_n$, $(c_{O3})_n$ of the at least one pollutant based on the digital pollution map **36** and at least one of the correlation factor $\alpha_n$ and the sensitivity factor $\beta$. Specifically, the processing module computes the corrected estimate $(C_{CO})_n$, $(C_{NO2})_n$, $(C_{SO2})_n$, $(C_{O3})_n$, of concentration of the at least one pollutant in the air at the corresponding locations and times based on the following formula/calculations:

$$(C_{CO})_n = \alpha_n * S11 * (c_{co})_n$$

$$(C_{NO2})_n = \alpha_n * S22 * (c_{NO2})_n$$

$$(C_{SO2})_n = \alpha_n * S33 * (c_{SO2})_n$$

$$(C_{O3})_n = \alpha_n * S22 * (c_{O3})_n$$

The processing module **40** further computes the Air Quality Index of the air based on corrected estimate $(C_{CO})_n$, $(C_{NO2})_n$, $(C_{SO2})_n$, $(C_{O3})_n$ of concentration of the at least one pollutant and at least one of actual measurement $(PM2.5)_n$, $(PM10)_n$ of the at least one air quality parameter and actual concentration measurement of the at least one pollutant measured by the at least one onboard sensor **20**. However, the present invention is not limited to any particular method or calculations for determining the Air Quality Index using the corrected estimate $(C_{CO})_n$, $(C_{NO2})_n$, $(C_{SO2})_n$, $(C_{O3})_n$ of concentration of the at least one pollutant and at least one of actual measurement $(PM2.5)_n$, $(PM10)_n$ of the at least one air quality parameter and actual concentration measurement of the at least one pollutant measured by the at least one onboard sensor **20**. Instead of corrected estimate of the pollutants mentioned above, the system **100** can determine corrected estimate of pollutants other than the above mentioned pollutants for determining the Air quality Index.

The system **100** for estimating Air Quality Index further includes a display module for displaying the Air Quality Index determined by the system **100** to the vehicle occupants. The display module is a display of at least one of the infotainment system disposed in the vehicle, a hand held, portable communication device that is communicably coupled to the vehicle via a communication network and carried by any of the vehicle occupants.

Further, is disclosed a heating ventilation and Air conditioning system in accordance with an embodiment of the present invention, wherein the heating ventilation and Air conditioning system controls operation of a re-circulation flap configured upstream of an evaporator of the heating ventilation and Air conditioning system and the re-circulation flap in turn selectively closes at least one of a fresh air supply conduit and a recirculation air supply conduit therein based on Air Quality Index determined by the system **100**. The heating ventilation and Air conditioning system also controls operation of other de-pollution devices based on Air Quality Index determined by the system **100**.

Also is disclosed a method for estimating Air Quality Index includes the steps of detecting location of a vehicle at any time by using a geo-location module **10,** measuring at least one of at least one air quality parameter and concentration of at least one pollutant associated with air outside a cabin of the vehicle at different locations and corresponding times by using the at least one on-board sensor **20,** determining by a pollution map module **30,** digital pollution map concentration $(pm2.5)_n$, $(pm10)_n$ of the at least one air quality parameter and digital pollution map concentration $(c_{CO})_n$, $(c_{NO2})_n$, $(c_{SO2})_n$, $(c_{O3})_n$ of the at least one pollutant in the air at the corresponding same locations and times based on a digital pollution map **36** stored in a remote server **34**, computing by a processing module **40** a correlation factor $\alpha_n$ and a sensitivity factor $\beta$, further computing by the processing module **40** a corrected estimate $(C_{CO})_n$, $(C_{NO2})_n$, $(C_{SO2})_n$, $(C_{O3})_n$ of concentration of the at least one pollutant in the air at the corresponding locations and times by using the digital pollution map concentration $(c_{CO})_n$, $(c_{NO2})_n$, $(c_{SO2})_n$, $(c_{O3})_n$ of the at least one pollutant based on the digital pollution map **36** and at least one of the correlation factor $\alpha_n$ and the sensitivity factor $\beta$ and computing the Air Quality Index of the air based on the corrected estimate $(C_{CO})_n$, $(C_{NO2})_n$, $(C_{SO2})_n$, $(C_{O3})_n$ of concentration of the at least one pollutant and at least one of actual measurement $(PM2.5)_n$, $(PM10)_n$ of the at least one air quality parameter and actual measurement of the at least one pollutant measured by the at least one onboard sensor **20**.

The method includes a step of computing the correlation factor $(\alpha_n)$ based on comparison of the at least one of actual measurement $(PM2.5)_n$, $(PM10)_n$ of the at least one air quality parameter and actual concentration measurement of at least one pollutant measured by the at least one onboard sensor **20** in close vicinity of the vehicle at different times to the corresponding digital pollution map concentration $(pm2.5)_n$, $(pm10)_n$ of the at least one air quality parameter and digital pollution map concentration $(c_{CO})_n$, $(c_{NO2})_n$, $(c_{SO2})_n$, $(c_{O3})_n$ of the at least one pollutant detected by the pollution map module **30** at the corresponding same locations and times based on big data over several segments of travel from the digital pollution map **36.**

Specifically, the method includes a step of computing the correlation factor $(\alpha_n)$ based on comparison of the actual measurement $(PM2.5)_n$, $(PM10)_n$ of the at least one air quality parameter measured by the onboard sensor **20** at different locations and times to the digital pollution map concentration $(pm2.5)_n$, $(pm10)_n$ of the same at least one air quality parameter detected by the pollution map module **30** at the corresponding same locations and times based on big data over several segments of travel stored in the digital pollution map **36**.

Alternatively, the method includes a step of computing the correlation factor $(\alpha_n)$ based on comparison of the at least one actual measurement of at least one pollutant such as at least one gas at different locations and times to the digital pollution map concentration of the same gas detected by the pollution map module **30** at the corresponding same locations and times based on big data over several segments of travel stored in the digital pollution map **36.**

Further, the method includes the step of computing the sensitivity factor $(\beta)$ based on comparison between actual pollution type detected to digital pollution map based pollution type detected by the pollution map module **30** by referring to the big data over several segments of travel from the digital pollution map **36,** the sensitivity factor $(\beta)$ is further tuned by said processing module **40** based on comparison between the corrected estimate $(C_{CO})_n$, $(C_{NO2})_n$, $(C_{SO2})_n$, $(C_{O3})_n$ of concentration of the pollutants and digital pollution map concentration $(c_{CO})_n$, $(c_{NO2})_n$, $(c_{SO2})_n$, $(c_{O3})_n$ of the pollutants based on big data over several segments of travel from the digital pollution map **36**.

Still further, the method includes the step of transmitting by at least one transceiver **32** of the pollution map module **30** of all the other vehicles configured with the system **100**, the corrected estimate $(C_{CO})_n$, $(C_{NO2})_n$, $(C_{SO2})_n$, $(C_{O3})_n$ of concentration of the at least one pollutant in the air and at least one of the actual measurement $(PM2.5)_n$, $(PM10)_n$ of the at least one air quality parameter and actual concentration measurement of the at least one pollutant measured by the at least one onboard sensor **20** at the various different locations and times in order to dynamically improve correlation and sensitivity factors based on big data storage and learning.

Furthermore, the method includes the step of displaying the Air Quality Index determined by the system **100** to the vehicle occupants on a display module of at least one of an infotainment system disposed in the vehicle, a hand held, portable communication device that is communicably coupled to the vehicle and carried by any of the vehicle occupants.

The above method is also applicable for determining any other indicator of air quality that involves determining accurate concentration of pollutants other than and not limiting to the gases based on digital pollution map concentration of the pollutants and particulate matter from pollution map stored in a remote server and actual measurement of the particulate matter and some of the pollutants such as gases , thereby eliminating the need of all the expensive pollutant sensors such as for example gas sensors.

Several modifications and improvement might be applied by the person skilled in the art to the system as defined above, as long as it comprises a geo-location module, at least one onboard sensor, a pollution map module and a processing module. The geo-location module detects location of the vehicle at any time. The at least one onboard sensor measures at least one of at least one air quality parameter and at least one pollutant at different locations and corresponding times. The pollution map module is configured with at least one transceiver that transmits location data related to the vehicle at any time to a remote server and receives digital pollution map concentration of the at least one air quality parameter and digital pollution map concentration of at least one pollutant in the air at the different locations and times based on a digital pollution map stored in the remote server. The processing module computes a correlation factor and a sensitivity factor. The processing module further computes a corrected estimate of concentration of the at least one pollutant in the air at the corresponding locations and times by using the digital pollution map concentration of the at least one pollutant based on big data over several segments of travel from the digital pollution map and at least one of the correlation factor and the sensitivity factor. The processing module further computes the Air Quality Index of the air based on corrected estimate of concentration of the at least one pollutant and concentration measurement of at least one of the at least one air quality parameter and at least one pollutant.

Obviously, numerous modifications and variations of the present invention are possible in light of the above teachings. It is therefore to be understood that the invention may be practiced otherwise than as specifically described herein.

In any case, the invention cannot and should not be limited to the embodiments specifically described in this document, as other embodiments might exist. The invention shall spread to any equivalent means and any technically operating combination of means.

## Claims

1. A system (100) for estimating Air Quality Index, the system (100) comprising:

   • a geo-location module (10) adapted to detect location of a vehicle at any time;
   • at least one onboard sensor (20) adapted to measure at least one of at least one air quality parameter, $(PM2.5)_n$, $(PM10)_n$ and concentration of at least one pollutant in close vicinity of the vehicle at different times;
   • a pollution map module (30) configured with at least one transceiver (32) adapted to transmit location and time data related to the vehicle at said different locations and corresponding times to a remote server (34) and receive digital pollution map concentration $(pm2.5)_n$, $(pm10)_n$ of said at least one air quality parameter and digital pollution map concentration $(c_{CO})_n$, $(c_{NO2})_n$, $(c_{SO2})_n$, $(c_{O3})_n$ of said at least one pollutant in the air at the corresponding same locations and times based on a digital pollution map (36) stored in said remote server (34); and
   • a processing module (40) adapted to compute a correlation factor $(\alpha_n)$ and a sensitivity factor $(\beta)$, said processing module (40) further adapted to compute a corrected estimate $(C_{CO})_n$, $(C_{NO2})_n$, $(C_{SO2})_n$, $(C_{O3})_n$ of concentration of said at least one pollutant in the air at the corresponding locations and times by using said digital pollution map concentration $(c_{CO})_n$, $(c_{NO2})_n$, $(c_{SO2})_n$, $(c_{O3})_n$ and said at least one of said correlation factor $(\alpha_n)$ and said sensitivity factor $(\beta)$, said processing module (40) still further adapted to compute the Air Quality Index of the air based on said corrected estimate $(C_{CO})_n$, $(C_{NO2})_n$, $(C_{SO2})_n$, $(C_{O3})_n$ of concentration of said at least one pollutant and at least one of actual measurement $(PM2.5)_n$, $(PM10)_n$ of said at least one air quality parameter and actual concentration measurement of said at least one pollutant by said at least one onboard sensor (20).

2. The system (100) for estimating Air Quality Index as claimed in any of the preceding claims, wherein said at least one onboard sensor (20) is at least one outside embedded sensor configured outside the vehicle and adapted to measure at least one of said at least one air quality parameter, $(PM2.5)_n$, $(PM10)_n$ and concentration of at least one pollutant.

3. The system (100) for estimating Air Quality Index as claimed in any of the preceding claims, wherein said pollution map module (30) is adapted to receive digital pollution map concentration for every available pollutants PM2.5, PM10, Carbon monoxide (CO), Nitrogen dioxide (NO2), Sulfur dioxide (SO2), Ozone (O3), and other pollutants and/or pollens based on big data over several segments of travel from said digital pollution map (36) stored in said remote server (34).

4. The system (100) estimating Air Quality Index as claimed in any of the preceding claims, wherein said processing module (40) is adapted to compute said correlation factor ($\alpha_n$) based on comparison of said at least one of actual measurement $(PM2.5)_n$, $(PM10)_n$ of said at least one air quality parameter and actual concentration measurement of at least one pollutant measured by said onboard sensor (20) at different locations and times to said corresponding digital pollution map concentration $(pm2.5)_n$, $(pm10)_n$ of said at least one air quality parameter and digital pollution map concentration $(c_{CO})_n$, $(c_{NO2})_n$, $(c_{SO2})_n$, $(c_{O3})_n$ of said at least one pollutant detected by said pollution map module (30) at the corresponding same locations and times based on big data over several segments of travel from said digital pollution map (36).

5. The system (100) estimating Air Quality Index as claimed in any of the preceding claims, wherein said processing module (40) is further adapted to compute said sensitivity factor ($\beta$) based on comparison between actual pollution type detected to digital pollution map based pollution type detected by said pollution map module (30) by referring to big data over several segments of travel from said digital pollution map (36), the processing module (40) is further adapted to tune the sensitivity factor ($\beta$) based on comparison between said corrected estimate $(C_{CO})_n$, $(C_{NO2})_n$, $(C_{SO2})_n$, $(C_{O3})_n$ of concentration of the pollutants and digital pollution map concentration $(c_{CO})_n$, $(c_{NO2})_n$, $(c_{SO2})_n$, $(c_{O3})_n$ of the pollutants based on big data over several segments of travel from said digital pollution map (36).

6. A method for estimating Air Quality Index comprising the steps of :

   • detecting location of a vehicle at any time by using a geo-location module (10);
   • measuring at least one of at least one air quality parameter, $(PM2.5)_n$, $(PM10)_n$ and concentration of at least one pollutant in close vicinity of the vehicle at different times by using at least one on-board sensor (20);
   • determining by a pollution map module (30), digital pollution map concentration $(pm2.5)_n$, $(pm10)_n$ of said at least one air quality parameter and digital pollution map concentration $(c_{CO})_n$, $(c_{NO2})_n$, $(c_{SO2})_n$, $(c_{O3})_n$ of said at least one pollutant in the air at the corresponding same locations and times based on a digital pollution map (36) stored in a remote server (34);
   • computing by a processing module (40) a correlation factor ($\alpha_n$) and a sensitivity factor ($\beta$), further computing by said processing module (40) a corrected estimate $(C_{CO})_n$, $(C_{NO2})_n$, $(C_{SO2})_n$, $(C_{O3})_n$ of concentration of said at least one pollutant in the air at the corresponding locations and times by using said digital pollution map concentration $(c_{CO})_n$, $(c_{NO2})_n$, $(c_{SO2})_n$, $(c_{O3})_n$ of said at least one pollutant and at least one of said correlation factor ($\alpha_n$) and said sensitivity factor ($\beta$); and
   • computing the Air Quality Index of the air based on said corrected estimate $(C_{CO})_n$, $(C_{NO2})_n$, $(C_{SO2})_n$, $(C_{O3})_n$ of concentration of said at least one pollutant and at least one of actual measurement $(PM2.5)_n$, $(PM10)_n$ of said at least one air quality parameter and actual concentration measurement of said at least one pollutant measured by said at least one onboard sensor (20).

7. The method for estimating Air Quality Index as claimed in claim 6, wherein said method comprising a step of computing said correlation factor ($\alpha_n$) based on comparison of said at least one of actual measurement $(PM2.5)_n$, $(PM10)_n$ of said at least one air quality parameter and actual concentration measurement of at least one pollutant measured by said onboard sensor (20) in close vicinity of the vehicle at different times to said corresponding digital pollution map concentration $(pm2.5)_n$, $(pm10)_n$ of said at least one air quality parameter and digital pollution map concentration $(c_{CO})_n$, $(c_{NO2})_n$, $(c_{SO2})_n$, $(c_{O3})_n$ of said at least one pollutant detected by said pollution map module (30) at the corresponding same locations and times based on big data over several segments of travel from said digital pollution map (36).

8. The method for estimating Air Quality Index as claimed in claim 6 or 7, further comprising a step of computing said sensitivity factor ($\beta$) based on comparison between actual pollution type detected to digital pollution map based pollution type detected by said pollution map module (30) by referring to big data over several segments of travel from said digital pollution map (36), said sensitivity factor ($\beta$) is further tuned by said processing module (40) based on comparison between said corrected estimate $(C_{CO})_n$, $(C_{NO2})_n$, $(C_{SO2})_n$, $(C_{O3})_n$ of concentration of the pollutants and digital pollution map concentration $(c_{CO})_n$, $(c_{NO2})_n$, $(c_{SO2})_n$, $(c_{O3})_n$ of the pollutants based on big data over several segments of travel from said digital pollution map (36).

9. The method for estimating Air Quality Index as claimed in claim 6 to 8, wherein said sensitivity factor ($\beta$) is either one of a diagonal matrix and a full matrix of $S_{i,j}$, wherein $i,j$ are pollutants for Air Quality Index (AQI) calculation, $S_{i,j}$ are always corrected by machine learning based on the big data.

DIGITAL POLLUTION MAP

36

34

32

100

20

ONBOARD SENSOR

10

GEO-LOCATION MODULE

at least one of $(PM2.5)_n$, $(PM10)_n$ and concentration of at least one pollutant such as gas

30

40

POLLUTION MAP MODULE

$(pm2.5)_n$, $(pm10)_n$, $(c_{co})_n$, $(c_{NO2})_n$, $(c_{SO2})_n$, $(c_{O3})_n$

PROCESSING MODULE COMPUTES $\alpha_n$, $\beta_n$ AND CORRECTED ESTIMATE OF CONCENTRATION OF GASES BASED ON BELOW EQUATION:

AIR QUALITY INDEX

$$\begin{bmatrix} (C_{CO})_n \\ (C_{NO2})_n \\ (C_{SO2})_n \\ (C_{O3})_n \end{bmatrix} = \underbrace{\frac{(PM2.5)_n\ (PM10)_n}{(pm2.5)_n\ (pm10)_n}}_{\alpha_n} \underbrace{\begin{bmatrix} S11 & 0 & 0 & 0 \\ 0 & S22 & 0 & 0 \\ 0 & 0 & S33 & 0 \\ 0 & 0 & 0 & S44 \end{bmatrix}}_{\beta} \begin{bmatrix} (c_{CO})_n \\ (c_{NO2})_n \\ (c_{SO2})_n \\ (c_{O3})_n \end{bmatrix}$$

FIG. 1

36

34

10

30

GEO-LOCATION MODULE

DIGITAL POLLUTION MAP

POLLUTION MAP MODULE

$(pm2.5)_0$ $(pm2.5)_1$ $(pm2.5)_2$ $(pm2.5)_3$ $(pm2.5)_4$ $(pm2.5)_5$ $(pm2.5)_6$ $(pm2.5)_7$ $(pm2.5)_8$ $(pm2.5)_9$

$(pm10)_0$ $(pm10)_1$ $(pm10)_2$ $(pm10)_3$ $(pm10)_4$ $(pm10)_5$ $(pm10)_6$ $(pm10)_7$ $(pm10)_8$ $(pm10)_9$

$(C_{co})_0$ $(C_{co})_1$ $(C_{co})_2$ $(C_{co})_3$ $(C_{co})_4$ $(C_{co})_5$ $(C_{co})_6$ $(C_{co})_7$ $(C_{co})_8$ $(C_{co})_9$

$(c_{NO2})_0$ $(c_{NO2})_1$ $(c_{NO2})_2$ $(c_{NO2})_3$ $(c_{NO2})_4$ $(c_{NO2})_5$ $(c_{NO2})_6$ $(c_{NO2})_7$ $(c_{NO2})_8$ $(c_{NO2})_9$

$(c_{SO2})_0$ $(c_{SO2})_1$ $(c_{SO2})_2$ $(c_{SO2})_3$ $(c_{SO2})_4$ $(c_{SO2})_5$ $(c_{SO2})_6$ $(c_{SO2})_7$ $(c_{SO2})_8$ $(c_{SO2})_9$

$(c_{O3})_0$ $(c_{O3})_1$ $(c_{O3})_2$ $(c_{O3})_3$ $(c_{O3})_4$ $(c_{O3})_5$ $(c_{O3})_6$ $(c_{O3})_7$ $(c_{O3})_8$ $(c_{O3})_9$

0  1  2  3  4  5  6  7  8  9

$(PM2.5)_0$ $(PM2.5)_9$

$(PM10)_0$ Concentration of at least one pollutant, gas $(PM10)_9$

ONBOARD SENSOR

20

$$\begin{bmatrix} (C_{CO})_n \\ (C_{NO2})_n \\ (C_{SO2})_n \\ (C_{O3})_n \end{bmatrix} = \underbrace{\frac{(PM2.5)_n \, (PM10)_n}{(pm2.5)_n \, (pm10)_n}}_{\alpha_n} \underbrace{\begin{bmatrix} S11 & 0 & 0 & 0 \\ 0 & S22 & 0 & 0 \\ 0 & 0 & S33 & 0 \\ 0 & 0 & 0 & S44 \end{bmatrix}}_{\beta} \begin{bmatrix} (c_{CO})_n \\ (c_{NO2})_n \\ (c_{SO2})_n \\ (c_{O3})_n \end{bmatrix}$$

PROCESSING MODULE

40

FIG. 2

Europäisches
Patentamt

European
Patent Office

Office européen
des brevets

# EUROPEAN SEARCH REPORT

Application Number

EP 18 19 7001

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/202824 A1 (BORREL HERVE [US]) 19 July 2018 (2018-07-19) * paragraph [0017] - paragraph [0026] * * paragraph [0037] - paragraph [0046] * * paragraph [0061]; figures 3,5 * | 1-9 | INV.<br>G06Q10/06<br>G01N33/00 |
| X | WILL HEDGECOCK ET AL: "Dissemination and presentation of high resolution air pollution data from mobile sensor nodes", 20100415; 20100415 - 20100417, 15 April 2010 (2010-04-15), pages 1-6, XP058103269, DOI: 10.1145/1900008.1900019 ISBN: 978-1-4503-0064-3 * the whole document * | 1-9 | |

TECHNICAL FIELDS
SEARCHED    (IPC)

G06Q
G01N

The present search report has been drawn up for all claims

| Place of search | Date of completion of the search | Examiner |
|---|---|---|
| The Hague | 19 October 2018 | Moynihan, Maurice |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
...................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 18 19 7001

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2018

| Patent document cited in search report | Publication date | Patent family member(s) | Publication date |
|---|---|---|---|
| US 2018202824 A1 | 19-07-2018 | NONE | |

EPO FORM P0459

For more details about this annex : see Official Journal of the European Patent Office, No. 12/82